# EUROPÄISCHE PATENTSCHRIFT

(11) **EP 1 365 678 B1**
(45) Veröffentlichungstag und Bekanntmachung des Hinweises auf die Patenterteilung: **25.03.2009**
(21) Anmeldenummer: 02722081.3
(22) Anmeldetag: 15.02.2002
(51) Int. Cl.: A61B 1/267, A61M 16/08

(54) **VERBINDUNGSSTÜCK FÜR EINEN DOPPELLUMENTUBUS MIT ZWEI EINZELLUMENTUBEN**
CONNECTING PIECE FOR A DOUBLE LUMEN TUBE WITH TWO INDIVIDUAL LUMEN TUBES
CONNECTEUR PERMETTANT DE RACCORDER DEUX TUBES A LUMIERE SIMPLE A UN TUBE A LUMIERE DOUBLE

(30) Priorität: 07.03.2001 UY 26609
(43) Veröffentlichungstag der Anmeldung: 03.12.2003
(73) Patentinhaber: ELECTROPLAST S.A., 12100 Montevideo (UY)
(72) Erfinder: RITTERSBERGER, Karl, Heinz, 64646 Heppenheim (DE); SCHAAF, Günter, 5078 Montevideo (UY)
(74) Vertreter: Helber, Friedrich
(86) Internationale Anmeldenummer: PCT/EP2002/001608
(87) Internationale Veröffentlichungsnummer: WO 2002/069786

(56) Entgegenhaltungen:
- DE-U- 29 613 610
- US-A- 4 919 651

## Beschreibung

Die Erfindung betrifft ein Verbindungsstück für einen Doppellumentubus mit zwei Einzellumentuben, insbesondere für den Einsatz im Tracheobronchialsystem. Dokument US-A-4 919 651 offenbart ein Verbindungsstück mit den Merkmalen des Oberbegriffs von Anspruch 1.

Solche auch als Endobronchialtuben bezeichneten Tuben oder Katheter, bei welchen an einem Ende eines Doppellumentubus wenigstens zwei Einzellumentuben jeweils an eines der Lumen des Doppellumentubus angeschlossen ist, werden beispielsweise als in die Luftröhre und/oder eine der Bronchien einzuführende Sonden oder Katheter verwendet und dienen beispielsweise zum ein- und/oder zweiseitigen Beatmen (ventilieren), zum Absaugen des Tracheobronchialsystems bzw. zum Einbringen von pharmazeutisch wirksamen oder der Diagnose bzw. Narkosemittel dienenden gasförmigen oder dünnflüssigen Medien. Auch zum Einführen von flexiblen, fiberoptischen Bronchoskopen zur Lagekontrolle der Doppellumentuben, für Diagnosezwecke oder operative Eingriffe werden solche Endobronchialtuben verwendet, die z.B. beim Einsatz in der Intubationsnarkose mit einem aufblasbaren Ballon zur Abdichtung der Trachea ausgestattet sein können. Von den jeweils an einem Lumen des Doppellumentubus angeschlosssenen Einzellumentuben wird der eine Tubus mit in die Luftröhre und der zweite weiter bis in den rechten oder linken Hauptbronchus eingeführt, wobei durch den jeweils in den getrennten Lumen vorgesehene aufblasbare Ballon die Lumen den Anforderungen entsprechend wahlweise verschlossen bzw. durchgängig gemacht werden können.

Ein Problem bei solchen Tuben ist der Übergangs- bzw. Verzweigungsbereich zwischen dem Doppellumentubus und den Einzellumentuben. Diese werden mittels in die jeweils anschließenden Lumen am zugeordneten Ende des Doppellumentubus bzw. des Einzellumentubus eingeschobene Verbindungstuben angeschlossen, was innerhalb der Lumen zu einer stufenartigen Querschnittsverengung führt. Das führt zu Turbulenzen in strömender Luft bzw. Gasen und behindert die Einführung und das Verschieben von Brochoskopen und führt zu Verletzungen an der empfindlichen Außenhaut des beweglichen Teils der Fiberoptik, was zur Zerstörung des teuren Instrumentes führen kann. Bestenfalls kann ein so verletztes Instrument einer kostenintensiven Reparatur zugeführt werden. Die faseroptische Bronchoskopie ist aber eine zunehmende Bedeutung gewinnende Diagnose- und Behandlungstechnik.

Der Erfindung liegt demgegenüber die Aufgabe zugrunde, die bekannten aus zwei an einem Ende eines Doppellumentubus angesetzten Einzellumentubus zusammengesetzten Endotrachialtuben so zu verbessern, dass die beobachtete Nachteile im Übergangs- oder Verzweigungsbereich mit Sicherheit vermieden sind und es so weder zu Beeinträchtigungen des Durchflusses bzw. der Durchströmung noch zu Beschädigungen von in das Tracheobronchialsystem einzuführenden Bronchoskopen kommen kann.

Erfindungsgemäß wird diese Aufgabe durch ein Verbindungsstück für einen Doppellumentubus mit zwei Einzellumentuben gelöst, welches einen sich in zwei Ausgangsanschlüssen für die Einzellumentuben verzweigenden Eingangsanschluss für den Doppellumentubus aufweist, wobei die lichten Durchlässe für den Doppellumentubus einerseits und die Ausgangsanschlüsse für die Einzellumentuben andererseits so ausgebildet sind, dass sie in der Querschnittsform ein im Wesentlichen stufenlosen kontinuierlichen Übergang in die Lumina der anzuschließenden Lumen sicherstellen.

In bevorzugter Ausgestaltung der Erfindung weist das Verbindungsstück dabei die Form eines Y auf, in welchem sich ein innerhalb des Eingangsanschlusses für den Doppellumentubus verlaufender Lumenabschnitt in zwei den Ausgangsanschlüssen für die Einzellumentuben zugeordnete Ausgangslumenabschnitte verzweigt.

Dabei ist es dann zweckmäßig, wenn der lichte Innenquerschnitt des zur Verbindung mit dem Doppellumentubus bestimmte Eingangsanschluss im Wesentlichen komplementär zur Außenwandung des Doppellumentubus in dessen Anschlussbereich an das Verbindungsstück geformt ist. Der Innenquerschnitt des Eingangsanschlusses kann dann in dem in der bestimmungsgemäßen Verbindungsstellung der Stirnkante des Doppellumentubus gegenüberstehenden Bereich einer einen umlaufenden Stufenanschlag bildenden Querschnittsverringerung versehen sein, welche so ausgebildet ist, dass der verbleibende Durchlassquerschnitt exakt dem Durchlassquerschnitt des Doppellumentubus entspricht. Wenn der Doppellumentubus dann bei der Montage der Einzellumentuben bis an den umlaufenden Stufenanschlag geschoben wird, erfolgt sozusagen eine automatische Ausrichtung und Zentrierung derart, dass die Querschnitte der Lumen im Doppellumentubus ohne stufenförmige Übergänge in die Durchlässe in den Ausgangsanschlüssen für die Einzellumen übergehen.

Wenn der innere Durchlassquerschnitt des Doppellumentubus in an sich bekannter Weise durch einen durchlaufenden mittleren Steg in zwei im Querschnitt etwa halbrunde Lumina unterteilt ist, empfiehlt es sich dann, im Übergangsbereich zwischen dem Anschluss für den Doppellumentubus zu den Anschlüssen für die Einzellumentuben im Verzweigungsbereich eine schlitzartige Vertiefung im Verbindungsstück vorzusehen, in welche dann ein verlängerter Abschnitt des Stegs des Doppellumentubus in der bestimmungsgemäßen Anschluss- bzw. Verbindungsstellung passend eingreift.

Auch die Ausgangsanschlüsse für die einlumigen Tuben weisen im Anschlussbereich mit den Tuben vorzugsweise einen Durchlassquerschnitt auf, welcher komplementär der Außenwandung der Einzellumentuben im Anschlussbereich entspricht. Die Montage der Einzellumentuben im Verbindungsstück erfolgt dann wieder durch Einschieben in den jeweils zugeordneten Ausgangsanschluss, wobei diese zweckmäßig wieder zur Begrenzung der Einschubtiefe der Einzellumentuben jeweils einen umlaufenden stufenförmigen Anschlag aufweisen, wobei dann wieder der lichte Durchlassquerschnitt des Verbindungsstücks im Anschlagbereich jeweils im Wesentlichen im Querschnitt des zugeordneten Lumen des anzuschließenden Einlumentubus entspricht.

Das Verbindungsstück wird vorzugsweise aus einem geeigneten thermoplastischen Kunststoffmaterial hergestellt, welches also die Herstellung im Spritzgussverfahren ermöglicht. Dabei kann dann auch ein Kunststoffmaterial mit elastomeren Eigenschaften Verwendung finden.

Alternativ ist auch eine Herstellung des Verbindungsstücks aus Metall möglich.

Von Vorteil kann es auch sein, wenn die ausgangsseitigen einlumigen Tuben als integrales Druck- oder Spritzgussteil zusammen mit dem Verbindungsstück hergestellt sind.

Die endgültige zug- und druckfeste sowie dichte Verbindung der anzuschließenden Tuben am Verbindungsstück kann auch dadurch gewährleistet werden, dass die Anschlussbereiche des Verbindungsstücks mit den anzuschließenden Tuben als Klebeverbindung ausgebildet sind.

Alternativ kann die Verbindung des Verbindungsstücks mit den anzuschließenden Tuben auch dadurch erfolgen, dass das Verbindungsstück direkt auf die Endabschnitte der anzuschließenden Tuben aufgespritzt wird.

Die Erfindung ist in der folgenden Beschreibung in Verbindung mit der Zeichnung näher erläutert, und zwar zeigt:
- Fig. 1: den grundsätzlichen Aufbau eines aus einem Doppel- lumentubus mit zwei an einem Ende angeschlossenen Einzellumentuben zusammengesetzten Endotracheal- tubus in bekannter Form in dreidimensionaler Darstel- lung;
- Fig. 2: den in dem in Fig. 1 innerhalb des strichpunktiert ein- geschlossenen Bereichs liegenden Endbereich eines Doppellumentubus und die zugehörigen zur Verbin- dung mit den anzuschließenden Einzellumentuben vor- gesehenen Verbindungstuben des bekannten Endotra- chealtubus in auseinander gezogener perspektivischer Darstellung;
- Fig. 3: einen unter Verwendung eines erfindungsgemäßen Verbindungsstücks aus einem Doppellumentubus und zwei angesetzten Einlumentuben hergestellten En- dotrachealtubus, der im Übrigen dem in Fig. 1 gezeig- ten Tubus entspricht;
- Fig. 4: den in Fig. 3 innerhalb des strichpunktierten Bereichs 4 gelegenen Verbindungsbereich des Doppellumentubus mit den angeschlossenen Einzellumentuben, wobei die rechte Hälfte des Doppellumentubus, der anschlie- ßende Bereich des Verbindungsstücks und der rechte abgehenden Einzellumentubus aufgeschnitten darge- stellt sind;
- Fig. 5: eine Darstellung des Verbindungsbereichs 4 in ausein- ander gezogenener Darstellung, wobei hier das Ver- bindungsstück teilweise aufgeschnitten dargestellt ist; und
- Fig. 6: eine der Fig. 4 entsprechende Ansicht, bei welcher zu- sätzlich an kurzen Anschlussabschnitten der aus dem Verbindungsstück abzweigenden Einzellumentuben Anschlussstutzen vorgesehen sind, an welchen an die spezielle Untersuchungsproblematik in der Länge an- gepasste Verlängerung der Einzellumentuben an- schließbar sind.

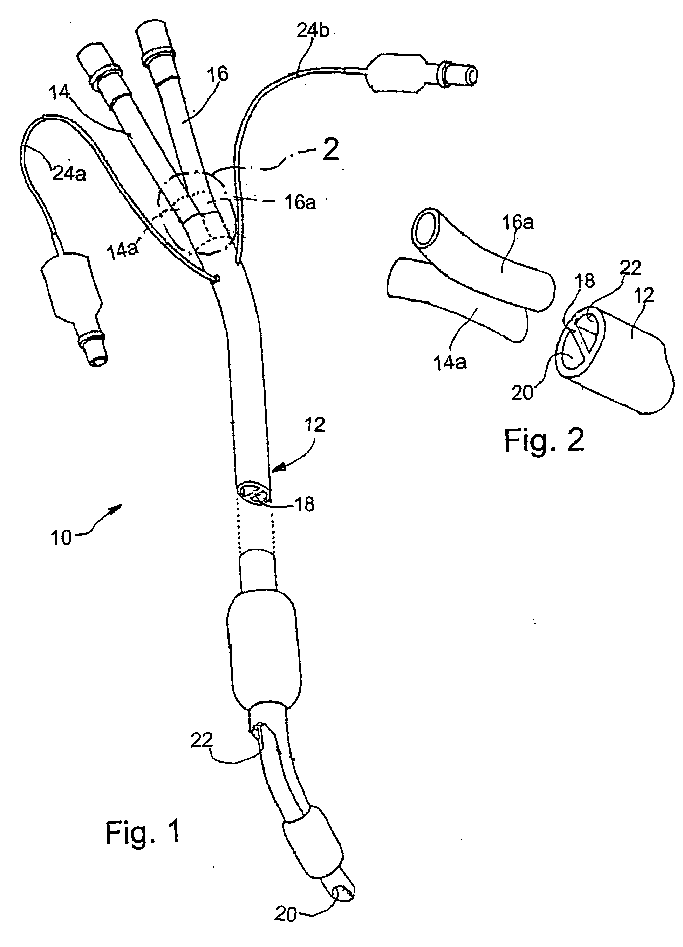

In Figur 1 ist ein für den Einsatz in Trachealbronchialsystemen vorgesehener, aus einem Doppellumentubus 12 und zwei an dessen proximalem Ende abzweigenden Einzellumentuben 14 und 16 zusammengesetzter Tubus 10 gemäß dem Stand der Technik gezeigt. Der Doppellumentubus 12 ist durch eine Trennwand 18 in zwei voneinander getrennte Lumen-Abschnitte 20 und 22 unterschiedlicher Länge unterteilt. Beide Abschnitte gehen von dem proximalen Endbereich des Doppellumentubus 12 aus, in welchem zwei gesonderte innere Rohr- oder Schlauchabschnitte 14a bzw. 16a in die voneinander getrennten Abschnitte 20 bzw. 22 des Doppellumentubus 12 eingeschoben sind. Ein Abschnitt ist vom distalen Endbereich aus bis in die Luftröhre und der andere weiter in den (rechten oder linken) Haupt-Bronchus geführt. Die gesonderten inneren Rohr- oder Schlauchabschnitte 14a sind mit ihren proximalen Endabschnitten in die Einzellumentuben 14 und 16 passend eingeführt. Das proximale Ende des Einzellumentubus 14 ist über den Schlauchabschnitt 14a dem Abschnitt 20 des Doppellumentubus 12 verbunden. In gleicher Weise ist der Einzellumentubus 16 über den Schlauchabschnitt 16a mit dem distalen Ende des Abschnitts 22 verbunden.

Der Tubus 10 weist zwei Sicherungs- und Verschluss-Ballons auf, von denen einer dem in die Luftröhre geführten Abschnitt und der andere dem den Bronchus geführten Abschnitt zugeordnet ist und welcher mittels zweier gesonderter Pumpventile 24a und 24b aufblasbar sind. Diese werden zur Regelung des Durchtritts von Luft, Gas und von medizinischen oder diagnostischen sowie therapeutischen Instrumenten verwendet, welche jeweils in Abhängigkeit von der durchzuführenden medizinischen oder chirurgischen Behandlung eingesetzt werden, wodurch eine geeignete Auswahl des zu behandelnden Pleuropulmonarbereichs ermöglicht wird.

Die beschriebene bekannte Ausgestaltung hat den Nachteil, dass die Einzellumentuben 14 und 16 auf den Außenseiten der Schlauchabschnitte 14a, 16a montiert werden. Als Folge hiervon entsteht innerhalb der Lumen im Verbindungsbereich eine Querschnittsverengung, welche zu Turbulenzen in durchströmender Luft führt und das Einführen und Vorschieben eines Bronchoskops erheblich behindert, wodurch diein neuerer Zeit zunehmend Bedeutung gewinnenden Diagnose- und Behandlungstechniken mittels faseroptischer Bronchoskopie erschwert wird. Darüber hinaus besteht Verletzungsgefahr an der beweglichen Spitze des Bronchoskopes, indem die hauchdünne Hüllse der Fiberoptik an der scharfen Kante der Engstelle zerstört wird.

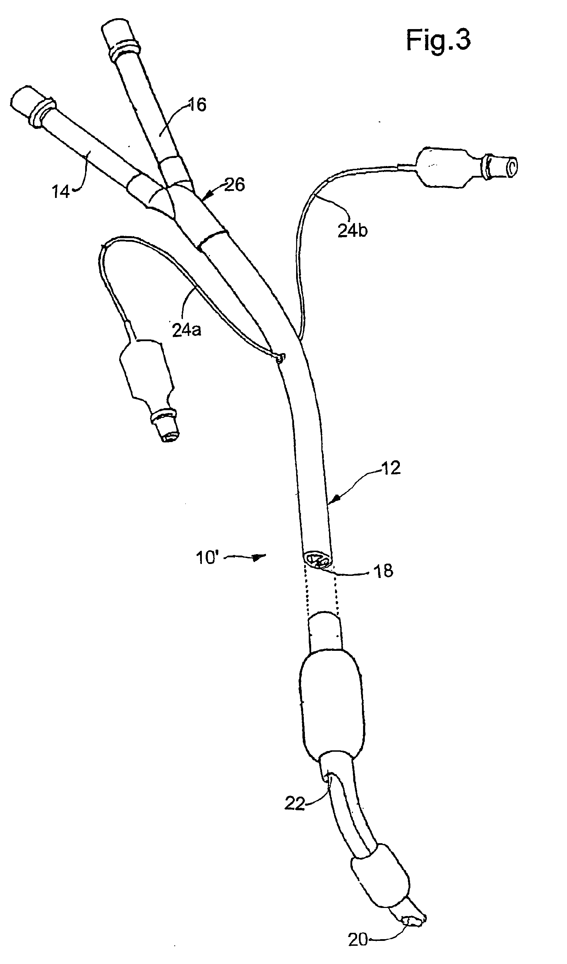

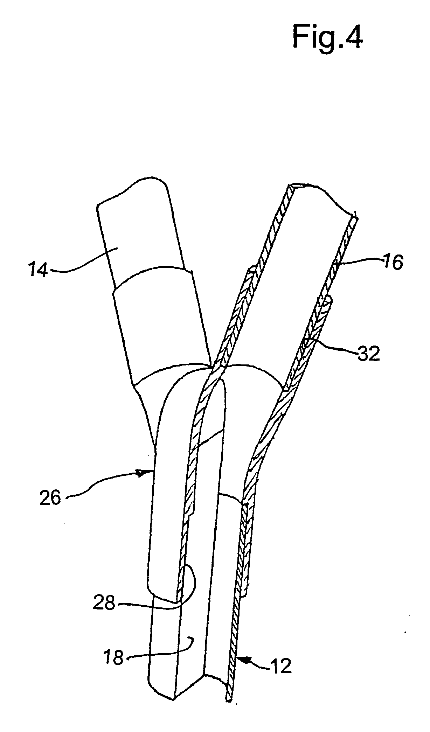

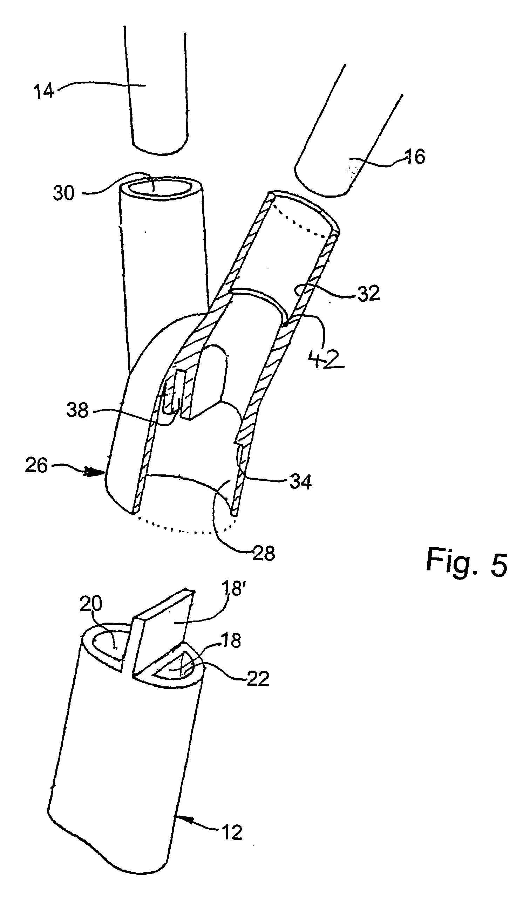

Figur 3 zeigt in einer der Darstellung der Figur 1 entsprechenden, im Trachealbronchialsystemen einsetzbare, aus einem Doppellumentubus 12 mit zwei von dessen proximalen Ende abzweigenden Einzellumentuben 14 und 16 zusammengesetzten Tubus 10', bei dem jedoch die Verbindung des Doppellumentubus 12 mit den Einzellumentuben 14 und 16 in erfindungsgemässer Weiterbildung über ein Verbindungsstück 26 erfolgt, welche so ausgebildet ist, dass die geschilderten Nachteile des bekannten Tubus 10 verminden sind. Eine nähere Beschreibung des Tubus 10' kann hier entfallen, weil er - bis auf das Verbindungsstück 26 - dem in Verbindung mit den Figuren 1 und 2 beschriebenen bekannten Tubus 10 entspricht und funktionellen gleichen Bauelementen der beiden Tuben 10 und 10' in der Figur 3 die gleichen Bezugszeichen zugeordnet sind wie in Figur 1. Es genügt daher, nachstehend anhand der Figuren 4 bis 6, die sich auf die spezielle Ausgestaltung des Verbindungsstücks 26 beziehenden Unterschiede der beiden Tuben 10 bzw. 10' zu erläutern.

Das Verbindungsstück 26 weist die äußere Gestalt eines Y auf, in welchem ein Eingangsanschluss 28 für den Doppellumentubus 12 sich intern in zwei Ausgangsanschlüsse 30, 32 für die Einzellumentuben 14 und 16 verzweigt.

Um einen im Wesentlichen stufenlosen kontinuierlichen Übergang der am Verbindungsstück 26 anzuschließenden Tuben 12 und 14 bzw. 16 sicherzustellen, ist der lichte Innenquerschnitt des zur Verbindung mit dem Doppellumentubus bestimmten Eingangsanschlusses 28 im Wesentlichen komplementär zur Außenwandung des Doppellumentubus 12 in dessen Anschlussbereich an das Verbindungsstück 26 geformt, wobei dieser komplementäre Bereich im Innern des Verbindungsstücks eine Querschnittsverringerung aufweist, welche einen Stufenanschlag 34 bildet. Die Bemessung der Querschnittsverringerung ist so getroffen, dass der verbleibende Durchlassquerschnitt exakt dem Durchlassquerschnitt des Doppellumentubus 12 entspricht, so dass also die Lumen-Abschnitte 20 bzw. 22 stufenlos in die Ausgangsanschlüsse 30, 32 für die Einzellumentuben 14, 16 verzweigen. Um die miteinander verbundenen Lumen 20 und 22 des Doppellumentubus innerhalb des Verbindungsstücks 26 gegeneinander abzudichten, weist die Trennwand 18 einen verlängerten, d.h. über die Stirnfläche des Doppellumentubus 12 etwas vortretenden Abschnitt 18' auf, welcher bei der Montage in einen zugeordneten Schlitz 38 im Verbindungsstück 26 passend eingeschoben wird.

Auch die Ausgangsanschlüsse 30 und 32 sind im Verbindungsbereich mit den Einzellumentuben 14 und 16 entsprechend dem Außendurchmesser dieser Einzellumentuben bemessen und erstrecken sich ins Innere des Verbindungsstücks bis zu einem jeweils umlaufenden stufenförmigen Anschlag 42, welcher die Einschubtiefe der Einzellumentuben in den jeweiligen Ausgangsanschluss 30 bzw. 32 bestimmt. Dabei ist wiederum der lichte Durchlassquerschnitt des Verbindungsstücks im vor dem Anschlag 42 liegenden Bereich so gewählt, dass der Durchlassquerschnitt des Verbindungsstücks 26 im Wesentlichen dem Querschnitt des zugeordneten Innenlumens des anzuschließenden Einlumentubus 14 bzw. 16 entspricht. Auch hier ist also wieder eine stufenförmige Einengung der Durchlässe im Anschlussbereich der Einzellumentuben 14, 16 vermieden.

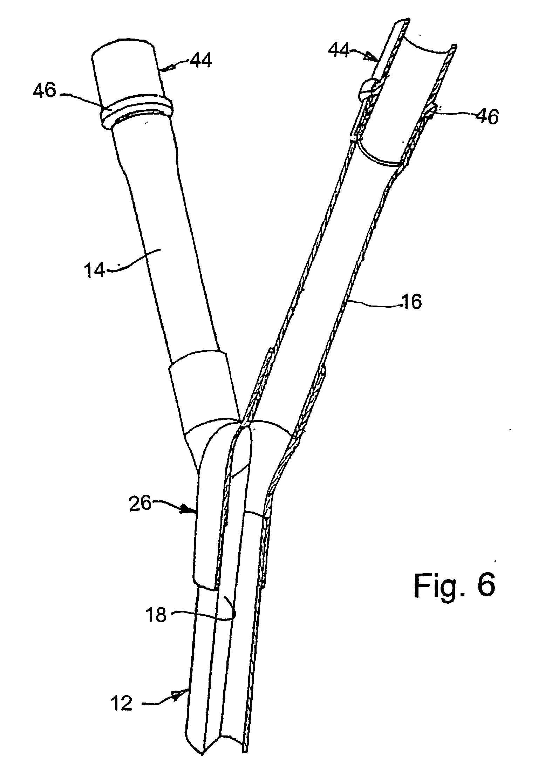

In Figur 6, welche im Übrigen im Wesentlichen der Figur 4 entspricht, ist noch die Möglichkeit einer stufenlosen Verlängerung der proximalen Enden der Einzellumentuben 14 und 16 durch jeweils einen Verbindungsstutzen 44 veranschaulicht, welcher praktisch aus einem kurzen Rohrabschnitt besteht, dessen lichter Innendurchmesser im Wesentlichen gleich dem lichten Innendurchmesser der Einzellumentuben 14 bzw. 16 ist. Ein außen umlaufender Ringvorsprung 46 stellt einen Anschlag für die proximalen Stirnflächen der Einzellumentuben dar, die beim Einschieben der Verbindungsstutzen im Einschubbereich der Verbindungsstutzen 44 elastisch aufgeweitet werden. Der lichte Innendurchmesser der Verbindungsstutzen entspricht also dem lichten Innendurchmesser des jeweils zugeordneten Einzellumentubus im nicht elastisch aufgeweiteten Zustand. Auf den jeweils freien Endbereich der Verbindungsstutzen 44 kann dann jeweils eine Verlängerung des zugeordneten Einzellumentubus 14 und/oder 16 aufgeschoben werden.

Es ist ersichtlich, dass im Rahmen des Erfindungsgedankens Abwandlungen und Weiterbildungen des beschriebenen Ausführungsbeispiels verwirklichbar sind, wie sie zum Beispiel bezüglich des verwendeten Materials in den Ansprüchen 9 bis 10 und bezüglich der Art und Weise der Verbindung der Tuben mit dem Verbindungsstutzen 26 in den Ansprüchen 11 bis 13 angegeben sind.

Wesentlich ist in jedem Fall, dass die Verbindung der Lumen-Abscnitte 20, 22 des Doppellumentubus 12 mit den Lumen der abzweigenden Einzellumentuben 14, 16 durch das Verbindungsstück 26 so erfolgt, dass an keiner Stelle des Durchlasses im Verbindungsstück 26 eine einengende Stufe entsteht, welche zu den genannten Nachteilen führt.

## Patentansprüche

1. Verbindungsstück (16) für einen Doppellumentubus (12) mit zwei Einzellumentuben (14; 16), insbesondere für den Einsatz im Tracheobronchialsystem,
wobei das Verbindungsstück (26) einen sich in zwei Ausgangsanschlüsse (30; 32) für die Einzellumentuben (14; 16) verzweigenden Eingangsanschluss (28) für den Doppellumentubus (12) aufweist, **dadurch gekennzeichnet, dass** die lichten Durchlässe für den Doppellumentubus (12) einerseits und die Ausgangsanschlüsse für die Einzellumentuben (14; 16) andererseits so ausgebildet sind, dass sie in der Querschnittsform einen im Wesentlichen stufenlosen kontinuierlichen Übergang in die Lumina der anzuschließenden Tuben sicherstellen.

2. Verbindungsstück nach Anspruch 1, **dadurch gekennzeichnet, dass** es die Form eines Y aufweist, in welchem sich ein innerhalb des Eingangsanschlusses (28) für den Doppellumentubus (12) verlaufender Lumenabschnitt in zwei, den Ausgangsanschlüssen (30; 32) für die Einzellumentuben (14; 16) zugeordnete Ausgangslumenabschnitte verzweigt.

3. Verbindungsstück nach Anspruch 1 oder 2, **dadurch gekennzeichnet, dass** der lichte Innenquerschnitt des zur Verbindung mit dem Doppellumentubus (12) bestimmten Eingangsanschlusses (28) im Wesentlichen komplementär zur Außenwandung des Doppellumentubus (12) in dessen Anschlussbereich an das Verbindungsstück (26) geformt ist.

4. Verbindungsstück nach Anspruch 3, **dadurch gekennzeichnet, dass** der Innenquerschnitt des Eingangsanschlusses (28) in dem in der bestimmungsgemäßen Verbindungsstellung der Stirnkante des Doppellumentubus (12) gegenüberstehenden Bereich mit einer einen umlaufenden Stufenanschlag (34) bildenden Querschnittsverringerung versehen ist, welche so ausgebildet ist, dass der verbleibende Durchlassquerschnitt exakt dem Durchlassquerschnitt des Doppellumentubus (12) entspricht.

5. Verbindungsstück nach einem der Ansprüche 1 bis 4, bei welchem der innere Durchlassquerschnitt des Doppellumentubus (12) durch einen durchlaufenden mittleren Steg (18) in zwei im Querschnitt halbrunde Lumina (20; 22) unterteilt ist, **dadurch gekennzeichnet, dass** im Übergangsbereich zwischen dem Anschluss für den Doppellumentubus (12) zu den Anschlüssen für die Einzellumentuben (14; 16) im Verzweigungsbereich eine schlitzartige Vertiefung (38) im Verbindungsstück (26) vorgesehen ist, in welche ein verlängerter Abschnitt (18') des Stegs (18) des Doppellumentubus (12) in der bestimmungsgemäßen Anschlussstellung passend eingreift.

6. Verbindungsstück nach einem der Ansprüche 1 bis 5, **dadurch gekennzeichnet, dass** die Ausgangsanschlüsse (30; 32) für die einlumigen Tuben (14; 16) im Anschlussbereich mit den Tuben einen lichten Durchlassquerschnitt aufweisen, welcher komplementär der Außenwandung der Einzellumentuben (14; 16) im Anschlussbereich entspricht.

7. Verbindungsstück nach Anspruch 6, **dadurch gekennzeichnet, dass** die Ausgangsanschlüsse (30; 32) für die Einzellumentuben (14; 16) zur Begrenzung von deren Einschubtiefe in die Ausgangsanschlüsse jeweils einen umlaufenden stufenförmigen Anschlag (42) aufweisen, und dass der lichte Durchlassquerschnitt des Verbindungsstücks (26) im Anschlagbereich jeweils im Wesentlichen dem Querschnitt des zugeordneten Lumen des anzuschließenden Innenlumentubus (14; 16) entspricht.

8. Verbindungsstück nach einem der Ansprüche 1 bis 7, **dadurch gekennzeichnet, dass** es aus einem thermoplastischen Material hergestellt ist.

9. Verbindungsstück nach einem der Ansprüche 1 bis 7, **dadurch gekennzeichnet, dass** es aus einem elastomeren Material hergestellt ist.

10. Verbindungsstück nach einem der Ansprüche 1 bis 7, **dadurch gekennzeichnet, dass** es aus Metall hergestellt ist.

11. Verbindungsstück nach einem der Ansprüche 1 bis 5, **dadurch gekennzeichnet, dass** die ausgangsseitigen einlumigen Tuben (14; 16) als integrale Druck- oder Spritzgußteile mit dem Verbindungsstück (26) hergestellt sind.

12. Verbindungsstück nach einem der Ansprüche 1 bis 11, **dadurch gekennzeichnet, dass** die Verbindung der Anschlussbereiche (28; 30; 32) des Verbindungsstücks (26) mit den anzuschließenden Tuben (12; 14; 16) als Klebeverbindung ausgebildet sind.

13. Verbindungsstück nach einem der Ansprüche 1 bis 9 und 11, **dadurch gekennzeichnet, dass** das Verbindungsstück (26) direkt auf die Endabschnitte der anzuschließenden Tuben (12; 14; 16) aufgespritzt ist.

## Claims

1. Connecting piece (16) for a double-lumen tube (12) with two single-lumen tubes (14; 16), in particular for use in the tracheobronchial system, wherein the connecting piece (26) has an input connection (28) for the double-lumen tube (12) which branches into two output connections (30; 32) for the single-lumen tubes (14; 16), **characterised in that** the clear passages for the double-lumen tube (12) on the one hand and the output connections for the single-lumen tubes (14; 16) on the other hand are constructed in such a way that in their cross-sectional shape they ensure a substantially step-free continuous transition into the lumina of the tubes to be connected.

2. Connecting piece as claimed in Claim 1, **characterised in that** it has the shape of a Y in which a lumen section which extends within the input connection (28) for the double-lumen tube (12) branches into two output lumen sections which are associated with the output connections (30; 32) for the single-lumen tubes (14; 16).

3. Connecting piece as claimed in Claim 1 or Claim 2, **characterised in that** the clear internal cross-section of the input connection (28) intended for connection to the double-lumen tube (12) is of substantially complementary shape to the outer wall of the double-lumen tube (12) in the region in which it is connected to the connecting piece (26).

4. Connecting piece as claimed in Claim 3, **characterised in that** the region of the internal cross-section of the input connection (28) which lies opposite the end edge of the double-lumen tube (12) in the proper connection position is provided with a narrowing of the cross-section which forms a circumferential step abutment (34) and is constructed in such a way that the remaining passage cross-section corresponds exactly to the passage cross-section of the double-lumen tube (12).

5. Connecting piece as claimed in any one of Claims 1 to 4, in which the internal passage cross-section of the double-lumen tube (12) is divided by a continuous central web (18) into two lumina (20; 22) which are approximately semi-circular in cross-section, **characterised in that** in the transition region between the connection for the double-lumen tube (12) and the connections for the single-lumen tubes (14; 16) a slot-like recess (38) is provided in the connection piece (26) in the branching region, wherein in the proper connection position a lengthened section (18') of the web (18) of the double-lumen tube (12) engages and fits into the said recess.

6. Connecting piece as claimed in any one of Claims 1 to 5, **characterised in that** the output connections (30; 32) for the single-lumen tubes (14; 16) have in the region in which they are connected to the tubes a clear passage cross-section which is complementary to the outer wall of the single-lumen tubes (14; 16) in the connection region.

7. Connecting piece as claimed in Claim 6, **characterised in that** the output connections (30; 32) for the single-lumen tubes (14; 16) each have a circumferential step-like abutment (42) to limit their depth of insertion into the output connections, and that the clear passage cross-section of the connecting piece (26) in the abutment region corresponds substantially to the cross-section of the associated lumen of the single-lumen tube (14; 16) to be connected.

8. Connecting piece as claimed in any one of Claims 1 to 7, **characterised in that** it is produced from a thermoplastic material.

9. Connecting piece as claimed in any one of Claims 1 to 7, **characterised in that** it is produced from an elastomeric material.

10. Connecting piece as claimed in any one of Claims 1 to 7, **characterised in that** it is produced from metal.

11. Connecting piece as claimed in any one of Claims 1 to 5, **characterised in that** the single-lumen tubes (14; 16) at the output end are produced as integral die-cast or injection-moulded parts with the connecting piece (26).

12. Connecting piece as claimed in any one of Claims 1 to 11, **characterised in that** the connection of the regions (28; 30; 32) where the connecting piece (26) is connected to the tubes (12; 14; 16) which are to be connected is constructed as a glued connection.

13. Connecting piece as claimed in any one of Claims 1 to 9 and 11, **characterised in that** the connecting piece (26) is injection-moulded directly onto the end sections of the tubes (12; 14; 16) to be connected.

## Revendications

1. Raccord (26) pour un tube à double lumière (12) avec deux tubes à simple lumière (14 ; 16), en particulier pour utilisation dans le système trachéo-bronchique, dans lequel le raccord (26) présente un raccordement d'entrée (28) pour le tube à double lumière (12) se ramifiant en deux raccordements de sortie (30 ; 32) pour les tubes à simple lumière (14 ; 16), **caractérisé en ce que** les passages intérieurs pour le tube à double lumière (12), d'une part, et les raccordements de sortie pour les tubes à simple lumière (14 ; 16), d'autre part, sont tels qu'ils garantissent dans la forme en section transversale une transition continue sensiblement progressive dans les lumières des tubes à raccorder.

2. Raccord selon la revendication 1, **caractérisé en ce qu'**il présente la forme d'un Y dans laquelle une section de lumière s'étendant à l'intérieur du raccordement d'entrée (28) pour le tube à double lumière (12) se ramifie en deux sections de lumière de sortie affectées aux raccordements de sortie (30 ; 32) pour les tubes à simple lumière (14 ; 16).

3. Raccord selon la revendication 1 ou 2, **caractérisé en ce que** la section transversale interne de passage du raccordement d'entrée (28) destiné au raccordement avec le tube à double lumière (12) est formée de manière sensiblement complémentaire avec la paroi externe du tube à double lumière (12) dans sa zone de raccordement au raccord (26).

4. Raccord selon la revendication 3, **caractérisé en ce que** la section transversale interne du raccordement d'entrée (28) présente, dans la zone opposée à l'arête frontale du tube à double lumière (12) dans la position de raccordement conforme, une réduction de section transversale qui forme une butée périphérique à paliers (34), réduction qui est conçue de sorte que la section transversale de passage restante corresponde exactement à la section transversale de passage du tube à double lumière (12).

5. Raccord selon l'une quelconque des revendications 1 à 4, dans lequel la section transversale de passage interne du tube à double lumière (12) est partagée par une nervure centrale continue (18) en deux lumières de section transversale semi-circulaire (20 ; 22), **caractérisé en ce que**, dans la zone de transition entre le raccordement pour le tube à double lumière (12) et les raccordements pour les tubes à simple lumière (14 ; 16) dans la zone de ramification, est prévu un évidement de type entaille (38) dans le raccord (26), dans lequel une section allongée (18') de la nervure (18) du tube à double lumière (12) s'engage de manière adaptée dans la position de raccordement conforme.

6. Raccord selon l'une quelconque des revendications 1 à 5, **caractérisé en ce que** les raccordements de sortie (30 ; 32) pour les tubes à simple lumière (14 ; 16) présentent, dans la zone de raccordement avec les tubes, une section transversale de passage intérieure qui correspond de manière complémentaire à la paroi externe des tubes à simple lumière (14 ; 16) dans la zone de raccordement.

7. Raccord selon la revendication 6, **caractérisé en ce que** les raccordements de sortie (30 ; 32) pour les tubes à simple lumière (14 ; 16) présentent pour limiter leur profondeur d'insertion dans les raccordements de sortie, respectivement, une butée périphérique par paliers (42) et **en ce que** la section transversale de passage intérieure du raccord (26) correspond, dans la zone de butée, respectivement, sensiblement à la section transversale de la lumière correspondante du tube à simple lumière à raccorder (14 ; 16).

8. Raccord selon l'une quelconque des revendications 1 à 7, **caractérisé en ce qu'**il est fabriqué en matériau thermoplastique.

9. Raccord selon l'une quelconque des revendications 1 à 7, **caractérisé en ce qu'**il est fabriqué en matériau thermoplastique.

10. Raccord selon l'une quelconque des revendications 1 à 7, **caractérisé en ce qu'**il est fabriqué en métal.

11. Raccord selon l'une quelconque des revendications 1 à 5, **caractérisé en ce que** les tubes à simple lumière (14 ; 16) côté sortie sont fabriqués sous la forme de pièces moulées par pression ou par injection d'une seule pièce avec le raccord (26).

12. Raccord selon l'une quelconque des revendications 1 à 11, **caractérisé en ce que** le raccordement des zones de raccordement (28 ; 30 ; 32) du raccord (26) avec les tubes à raccorder (12 ; 14 ; 16) se présente sous la forme d'un raccordement par collage

13. Raccord selon l'une quelconque des revendications 1 à 9 et 11, **caractérisé en ce que** le raccord (26) est directement injecté sur les sections d'extrémité des tubes à raccorder (12 ; 14 ; 16).
